# EUROPÄISCHE PATENTSCHRIFT

(11) **EP 2 872 494 B1**
(45) Veröffentlichungstag und Bekanntmachung des Hinweises auf die Patenterteilung: **15.06.2016**
(21) Anmeldenummer: 13734413.1
(22) Anmeldetag: 05.07.2013
(51) Int. Cl.: C07D 295/023

(54) **VERFAHREN ZUR HERSTELLUNG VON PYRROLIDIN**
PROCESS FOR THE PREPARATION OF PYRROLIDINE
PROCÉDÉ DE PRÉPARATION DE PYRROLIDINE

(30) Priorität: 13.07.2012 EP 12176375
(43) Veröffentlichungstag der Anmeldung: 20.05.2015
(73) Patentinhaber: BASF SE, 67056 Ludwigshafen am Rhein (DE)
(72) Erfinder: BOU CHEDID, Roland, 68159 Mannheim (DE); MELDER, Johann-Peter, 67459 Böhl-Iggelheim (DE); DOSTALEK, Roman, 67271 Neuleiningen (DE); PASTRE, Jörg, 64625 Bensheim (DE); TAN, Aik Meam, 67346 Speyer (DE)
(74) Vertreter: BASF IP Association
(86) Internationale Anmeldenummer: PCT/EP2013/064321
(87) Internationale Veröffentlichungsnummer: WO 2014/009292

(56) Entgegenhaltungen:
- EP-A1- 1 312 600
- WO-A1-2011/067199

## Beschreibung

Die vorliegende Erfindung betrifft ein Verfahren zur Herstellung von Pyrrolidin der Formel I 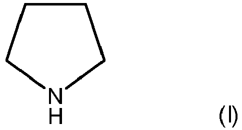 durch Umsetzung von 1,4-Butandiol (BDO) der Formel II 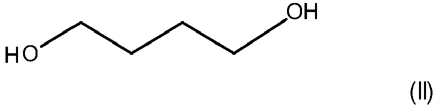 mit Ammoniak in Gegenwart von Wasserstoff und eines geträgerten, metallhaltigen Katalysators.

Pyrrolidin findet u.a. Verwendung als Zwischenprodukt und/oder Reagenz bei der Herstellung von Kraftstoffadditiven (US 3,275,554 A; DE 21 25 039 A und DE 36 11 230 A), Tensiden, Arznei- und Pflanzenschutzmitteln, Härtern für Epoxyharze, Katalysatoren für Polyurethane, und als Zwischenprodukt und/oder Reagenz bei der Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und Emulgatoren.

WO 03/051508 A1 (Huntsman Petrochemical Corp.) betrifft Verfahren zur Aminierung von Alkoholen unter Verwendung von spezifischen Cu/Ni/Zr/Sn - haltigen Katalysatoren, die in einer weiteren Ausgestaltung Cr statt Zr enthalten (siehe Seite 4, Zeilen 10-16). Die in dieser WO-Anmeldung beschriebenen Katalysatoren enthalten kein Aluminiumoxid und kein Kobalt.

WO 2008/006750 A1 (BASF AG) betrifft bestimmte Pb, Bi, Sn, Sb und/oder In - dotierte, zirkoniumdioxid-, kupfer-, nickel- und kobalthaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2009/080507 A1 (BASF SE) betrifft bestimmte Sn- und Co-dotierte, zirkoniumdioxid-, kupfer- und nickelhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2009/080506 A1 (BASF SE) beschreibt bestimmte Pb, Bi, Sn, Mo, Sb und/oder P - dotierte, zirkoniumdioxid-, nickel- und eisenhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt. Bevorzugt enthalten die Katalysatoren kein Cu und kein Co.

WO 2009/080508 A1 (BASF SE) lehrt bestimmte Pb, Bi, Sn und/oder Sb - dotierte, zirkoniumdioxid-, kupfer-, nickel-, kobalt- und eisenhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2011/067199 A1 (BASF SE) betrifft bestimmte aluminiumoxid-, kupfer-, nickel-, kobalt- und zinnhaltige Katalysatoren und ihre Verwendung in Verfahren zur Herstellung eines Amins aus einem primären oder sekundären Alkohol, Aldehyd und/oder Keton. Die Herstellung von Pyrrolidinen aus 1,4-Diolen wird auf Seite 22, Zeilen 13-15, und Seite 24, Zeilen 39-42, allgemein erwähnt.

WO 2011/157710 A1 (BASF SE) beschreibt die Herstellung bestimmter zyklischer tertiärer Methylamine, wobei man einen Aminoalkohol aus der Gruppe 1,4-Aminobutanol, 1,5-Aminopentanol, Aminodiglykol (ADG) bzw. Aminoethyl-ethanolamin, mit Methanol bei erhöhter Temperatur in Gegenwart eines kupferhaltigen Heterogenkatalysators in der Flüssigphase umsetzt.

WO 2012/049101 A1 (BASF SE) betrifft ein Verfahren zur Herstellung bestimmter zyklischer tertiärer Amine, indem man einen Aminoalkohol aus der Gruppe 1,4-Aminobutanol, 1,5-Aminopentanol, Aminodiglykol (ADG) bzw. Aminoethyl-ethanolamin, mit einem bestimmten primären oder sekundären Alkohol bei erhöhter Temperatur in Gegenwart eines kupferhaltigen Heterogenkatalysators in der Flüssigphase umsetzt.

DE 198 59 776 A1 (BASF AG) betrifft bestimmte Aminierungsverfahren unter Einsatz von Katalysator-Formkörpern, die sauerstoffhaltige Verbindungen des Titans und des Kupfers und metallisches Kupfer umfassen.

Der vorliegenden Erfindung lag die Aufgabe zugrunde, die Wirtschaftlichkeit bisheriger Verfahren zur Herstellung von Pyrrolidin der Formel I zu verbessern und einem Nachteil oder mehreren Nachteilen des Stands der Technik abzuhelfen. Es sollten Bedingungen gefunden werden, die technisch in einfacher Weise herzustellen sind und die es erlauben, das Verfahren mit hohem Umsatz, hoher Ausbeute, Raum-Zeit-Ausbeute (RZA), Selektivität bei gleichzeitig hoher mechanischer Stabilität des Katalysatorformkörpers und geringer 'Durchgehgefahr', durchzuführen.

[Raum-Zeit-Ausbeuten werden angegeben in ,Produktmenge / (Katalysatorvolumen • Zeit)' (kg / (l_{Kat.} • h)) und/oder,Produktmenge / (Reaktorvolumen • Zeit)' (kg / (l_{Reaktor} • h)].

Demgemäß wurde ein Verfahren zur Herstellung von Pyrrolidin der Formel I 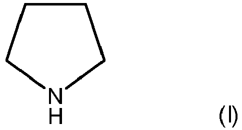 durch Umsetzung von 1,4-Butandiol (BDO) der Formel II 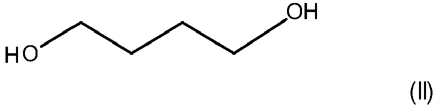 mit Ammoniak in Gegenwart von Wasserstoff und eines geträgerten, metallhaltigen Katalysators gefunden, welches dadurch gekennzeichnet ist, dass die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthält und man die Umsetzung in der Flüssigphase bei einem Absolutdruck im Bereich von 160 bis 220 bar, einer Temperatur im Bereich von 160 bis 230 °C, unter Einsatz von Ammoniak in einem Molverhältnis zu eingesetztem BDO von 5 bis 50 und in Gegenwart von 1,0 bis 4,5 Gew.-% Wasserstoff, bezogen auf die Menge an eingesetztem BDO, durchführt.

Insbesondere werden Katalysatoren, deren katalytisch aktive Masse vor deren Reduktion mit Wasserstoff im Bereich von
15 bis 80 Gew.-% sauerstoffhaltige Verbindungen des Aluminiums, berechnet als Al₂O₃,
1 bis 20 Gew.-% sauerstoffhaltige Verbindungen des Kupfers, berechnet als CuO,
5 bis 35 Gew.-% sauerstoffhaltige Verbindungen des Nickels, berechnet als NiO,
5 bis 35 Gew.-% sauerstoffhaltige Verbindungen des Kobalts , berechnet als CoO, und
0,2 bis 5,0 Gew.-% sauerstoffhaltige Verbindungen des Zinns, berechnet als SnO, enthält, im o. g. Aminierungsverfahren eingesetzt.

Das Verfahren kann kontinuierlich oder diskontinuierlich durchgeführt werden. Bevorzugt ist eine kontinuierliche Fahrweise.

In der Kreisgasfahrweise werden die Ausgangsstoffe (BDO, Ammoniak) in einem Kreisgasstrom verdampft und gasförmig dem Reaktor zugeführt. Die Edukte (BDO, Ammoniak) können auch als wässrige Lösungen verdampft und mit dem Kreisgasstrom auf das Katalysatorbett geleitet werden.

Bevorzugte Reaktoren sind Rohrreaktoren. Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Alternativ erfolgt die Umsetzung vorteilhaft in einem Rohrbündelreaktor oder in einer Monostranganlage. Bei einer Monostranganlage kann der Rohrreaktor, in dem die Umsetzung erfolgt, aus einer Hintereinanderschaltung mehrerer (z. B. zweier oder dreier) einzelner Rohrreaktoren bestehen. Optional ist hier vorteilhaft eine Zwischeneinspeisung von Feed (enthaltend das BDO und/oder Ammoniak und/oder H₂) und/oder Kreisgas und/oder Reaktoraustrag aus einem nachgeschalteten Reaktor möglich. Das Kreisgas enthält bevorzugt mindestens 10, besonders 50 bis 100, ganz besonders 80 bis 100, Vol.% H₂.

Die Kreisgasmenge liegt, im Fall eines H₂-Gehalts von 80 Vol.%, bevorzugt im Bereich von 10 bis 450 Nm³ / [m³ Katalysator (Schüttvolumen) • h], insbesondere im Bereich von 15 bis 450 Nm³ / [m³ Katalysator (Schüttvolumen) • h], weiter besonders im Bereich von 20 bis 400 Nm³ / [m³ Katalysator (Schüttvolumen) • h], ganz besonders im Bereich von 25 bis 400 Nm³ / [m³ Katalysator (Schüttvolumen) • h], z.B. 35 bis 360 Nm³ / [m³ Katalysator (Schüttvolumen) • h]. Bei einem anderen H₂-Gehalt ändern sich die o.g. Kreisgasmengen rechnerisch entsprechend, um die H₂-Menge (in Nm³ / [m³ Katalysator (Schüttvolumen) • h]) konstant zu halten. [Nm³ = Normkubikmeter = auf Normalbedingungen (20 °C, 1 bar abs.) umgerechnetes Volumen]. (Normaldruck = 1 bar abs.).

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.
In diesem Zusammenhang wird das oxidische Trägermaterial Aluminiumoxid (Al₂O₃) als zur katalytisch aktiven Masse gehörig gewertet.

Die Katalysatoren werden dergestalt eingesetzt, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z. B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o. g. Katalysatorträgermaterialien definiert und enthält im Wesentlichen die folgenden Bestandteile:
Aluminiumoxid (Al₂O₃), sauerstoffhaltige Verbindungen des Kupfers, Nickels und Kobalts und sauerstoffhaltige Verbindungen des Zinns.

Die Summe der o. g. Bestandteile der katalytisch aktiven Masse beträgt üblicherweise 70 bis 100 Gew.-%, bevorzugt 80 bis 100 Gew.-%, besonders bevorzugt 90 bis 100 Gew.-%, besonders > 95 Gew.-%, ganz besonders > 98 Gew.-%, insbesondere > 99 Gew.-%, z. B. besonders bevorzugt 100 Gew.-%.

Die katalytisch aktive Masse der erfindungsgemäßen und im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

Beispiele für solche Elemente bzw. deren Verbindungen sind:
Übergangsmetalle, wie Mn bzw. MnO₂, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Erdalkalimetalloxide, wie SrO; Erdalkalimetallcarbonate, wie MgCO₃, CaCO₃ und BaCO₃; Boroxid (B₂O₃).

Bevorzugt enthält die katalytisch aktive Masse der erfindungsgemäßen und im erfindungsgemäßen Verfahren eingesetzten Katalysatoren kein Rhenium, kein Ruthenium, kein Eisen und/oder kein Zink, jeweils weder in metallischer (Oxidationsstufe = 0) noch in einer ionischen (Oxidationsstufe ≠ 0), insbesondere oxidierten, Form.

Bevorzugt enthält die katalytisch aktive Masse der erfindungsgemäßen und im erfindungsgemäßen Verfahren eingesetzten Katalysatoren kein Silber und/oder Molybdän, jeweils weder in metallischer (Oxidationsstufe = 0) noch in einer ionischen (Oxidationsstufe ≠ 0), insbesondere oxidierten, Form.

In einer besonders bevorzugten Ausführungsform enthält die katalytisch aktive Masse der erfindungsgemäßen und im erfindungsgemäßen Verfahren eingesetzten Katalysatoren keine weitere katalytisch aktive Komponente, weder in elementarer (Oxidationsstufe = 0) noch in ionischer (Oxidationsstufe ≠ 0) Form.

In der besonders bevorzugten Ausführungsform ist die katalytisch aktive Masse nicht mit weiteren Metallen oder Metallverbindungen dotiert.
Bevorzugt sind jedoch aus der Metallgewinnung von Cu, Co, Ni, Sn herrührende übliche Begleit-Spurenelemente hiervon ausgenommen.

Bevorzugt enthält die katalytisch aktive Masse des Katalysators keine sauerstoffhaltigen Verbindungen des Siliziums und/oder des Zirkoniums.
Bevorzugt enthält die katalytisch aktive Masse des Katalysators keine sauerstoffhaltigen Verbindungen des Titans und/oder des Chroms.

Die katalytisch aktive Masse des Katalysators enthält vor dessen Reduktion mit Wasserstoff im Bereich von 0,2 bis 5,0 Gew.-%, besonders im Bereich von 0,4 bis 4,0 Gew.-%, weiter besonders im Bereich von 0,6 bis 3,0 Gew.-%, weiter besonders bevorzugt im Bereich von 0,7 bis 2,5 Gew.-%, sauerstoffhaltige Verbindungen des Zinns, berechnet als SnO.

Die katalytisch aktive Masse des Katalysators enthält vor dessen Reduktion mit Wasserstoff bevorzugt im Bereich von 5,0 bis 35 Gew.-%, besonders im Bereich von 10 bis 30 Gew.-%, weiter besonders im Bereich von 12 bis 28 Gew.-%, ganz besonders 15 bis 25 Gew.-%, sauerstoffhaltige Verbindungen des Kobalts, berechnet als CoO.

Die katalytisch aktive Masse des Katalysators enthält vor dessen Reduktion mit Wasserstoff weiterhin bevorzugt im Bereich von
15 bis 80 Gew.-%, besonders 30 bis 70 Gew.-%, weiter besonders 35 bis 65 Gew.-%, sauerstoffhaltige Verbindungen des Aluminiums, jeweils berechnet als Al₂O₃,
1 bis 20 Gew.-%, besonders 2 bis 18 Gew.-%, weiter besonders 5 bis 15 Gew.-%, sauerstoffhaltige Verbindungen des Kupfers, jeweils berechnet als CuO, und
5 bis 35 Gew.-%, besonders 10 bis 30 Gew.-%, weiter besonders 12 bis 28 Gew.-%, ganz besonders 15 bis 25 Gew.-%, sauerstoffhaltige Verbindungen des Nickels, jeweils berechnet als NiO.

Das Molverhältnis von Nickel zu Kupfer beträgt bevorzugt größer 1, besonders bevorzugt größer 1,2, weiter besonders bevorzugt im Bereich von 1,8 bis 8,5.

Die BET-Oberfläche (ISO 9277:1995) der erfindungsgemäßen und im erfindungsgemäßen Verfahren eingesetzten Katalysatoren liegt bevorzugt im Bereich von 30 bis 250 m²/g, besonders im Bereich von 90 bis 200 m²/g, weiter besonders im Bereich von 130 bis 190 m²/g, (jeweils vor der Reduktion mit Wasserstoff). Diese Bereiche werden insbesondere durch Calciniertemperaturen bei der Katalysatorherstellung im Bereich von 400 bis 600 °C, besonders 420 bis 550 °C, (vgl. unten) erzielt.

Zur Herstellung der im erfindungsgemäßen Verfahren verwendeten Katalysatoren sind verschiedene Verfahren möglich. Sie sind beispielsweise durch Peptisieren pulvriger Mischungen der Hydroxide, Carbonate, Oxide und/oder anderer Salze der Komponenten mit Wasser und nachfolgendes Extrudieren und Tempern (Wärmebehandlung) der so erhaltenen Masse erhältlich.

Bevorzugt werden zur Herstellung der erfindungsgemäßen Katalysatoren Fällungsmethoden angewandt. So können sie beispielsweise durch eine gemeinsame Fällung der Nickel-, Kobalt-, Kupfer- und Sn-Komponenten aus einer diese Elemente enthaltenden, wässrigen Salzlösung mittels Basen in Gegenwart einer Aufschlämmung einer schwerlöslichen, sauerstoffhaltigen Aluminiumverbindung und anschließendes Waschen, Trocknen und Calcinieren des erhaltenen Präzipitats erhalten werden. Als schwerlösliche, sauerstoffhaltige Aluminiumverbindungen können beispielsweise Aluminiumoxid, Aluminiumoxidhydrat, Aluminiumphosphate, -borate und - silikate Verwendung finden. Die Aufschlämmungen der schwerlöslichen Aluminiumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Aluminiumverbindungen aus wässrigen Aluminiumsalzlösungen mittels Basen erhalten.

Bevorzugt werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Base, - beispielsweise Natriumcarbonat, Natriumhydroxid, Kaliumcarbonat oder Kaliumhydroxid - versetzt, bis die Fällung vollständig ist. Es kann auch mit Alkalimetall-freien Basen wie Ammoniak, Ammoniumcarbonat, Ammoniumhydrogencarbonat, Ammoniumcarbamat, Ammoniumoxalat, Ammoniummalonat, Urotropin, Harnstoff, etc. gearbeitet werden. Die Art der verwendeten Salze ist im Allgemeinen nicht kritisch:
Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im Allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d. h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden zu den erfindungsgemäßen Katalysatoren wie üblich weiterverarbeitet. Zunächst werden die Niederschläge gewaschen. Über die Dauer des Waschvorgangs und über die Temperatur und Menge des Waschwassers kann der Gehalt an Alkalimetall, das durch die als Fällungsmittel eventuell verwendete (Mineral)base zugeführt wurde, beeinflusst werden. Im Allgemeinen wird durch Verlängerung der Waschzeit oder Erhöhung der Temperatur des Waschwassers der Gehalt an Alkalimetall abnehmen. Nach dem Waschen wird das Fällgut im Allgemeinen bei 80 bis 200 °C, vorzugsweise bei 100 bis 150 °C, getrocknet und danach calciniert. Die Calcinierung wird im Allgemeinen bei Temperaturen zwischen 300 und 800 °C, vorzugsweise bei 400 bis 600 °C, insbesondere bei 420 bis 550 °C ausgeführt.

Die erfindungsgemäßen Katalysatoren können auch durch Tränkung von Aluminiumoxid (Al₂O₃), das beispielsweise in Form von Pulver oder Formkörpern, wie Strängen, Tabletten, Kugeln oder Ringen, vorliegt, hergestellt werden.

Das Aluminiumoxid wird beispielsweise in der amorphen, gamma-, theta- und/oder delta- Form, als Aluminiumoxohydroxid (Böhmit), bevorzugt in der amorphen Form eingesetzt.

Die Herstellung von Formkörpern kann nach den üblichen Verfahren erfolgen.

Die Tränkung erfolgt ebenfalls nach den üblichen Verfahren, wie z. B. in A. B. Stiles, Catalyst Manufacture - Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und optional calciniert.

Die Tränkung kann nach der sogenannten "incipient wetness"-Methode erfolgen, bei der das Aluminiumoxid entsprechend seiner Wasseraufnahmekapazität maximal bis zur Sättigung mit der Tränklösung befeuchtet wird. Die Tränkung kann aber auch in überstehender Lösung erfolgen.
Bei mehrstufigen Tränkverfahren ist es zweckmäßig, zwischen einzelnen Tränkschritten zu trocknen und optional zu calcinieren. Die mehrstufige Tränkung ist vorteilhaft besonders dann anzuwenden, wenn das Aluminiumoxid mit einer größeren Metallmenge beaufschlagt werden soll.
Zur Aufbringung der Metallkomponenten auf das Aluminiumoxid kann die Tränkung gleichzeitig mit allen Metallsalzen oder in beliebiger Reihenfolge der einzelnen Metallsalze nacheinander erfolgen.

Anschließend werden die durch Tränkung hergestellten Katalysatoren getrocknet und bevorzugt auch calciniert, z. B. bei den bereits oben angegebenen Calciniertemperaturbereichen.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Presse zu Formlingen, z. B. Tabletten, verpresst und tempert. Die Tempertemperaturen entsprechen dabei bevorzugt den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d. h. insbesondere als Oxide und Mischoxide.

Die z. B. wie oben beschrieben hergestellten Katalysatoren werden als solche gelagert und ggf. gehandelt. Vor ihrem Einsatz als Katalysatoren werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden.

Zur Vorreduktion werden die Katalysatoren zunächst bei bevorzugt 150 bis 200 °C über einen Zeitraum von z. B. 12 bis 20 Stunden einer Stickstoff-Wasserstoff-Atmosphäre ausgesetzt und anschließend noch bis zu ca. 24 Stunden bei bevorzugt 200 bis 400 °C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Das erfindungsgemäße Verfahren wird bevorzugt kontinuierlich durchgeführt, wobei der Katalysator bevorzugt als Festbett im Reaktor angeordnet ist. Dabei ist sowohl eine Anströmung des Katalysatorfestbetts von oben als auch von unten möglich.

Der Ammoniak wird in der 5- bis 50-fachen molaren Menge, bevorzugt 11- bis 45-fachen molaren Menge, weiter bevorzugt 13- bis 40-fachen molaren Menge, besonders 15- bis 38-fachen molaren Menge, insbesondere in der 17- bis 36-fachen molaren Menge, in der ≥20- bis 36-fachen molaren Menge, z.B. 22- bis 35-fachen molaren Menge, jeweils bezogen auf das eingesetzte BDO, eingesetzt.

Der Ammoniak kann als wässrige Lösung, besonders als 30 bis 100 Gew.-%ige wässrige Lösung, z.B. 50 bis 90 Gew.-%ige wässrige Lösung, eingesetzt werden. Er wird bevorzugt ohne weiteres Lösungsmittel (Druckgas, Reinheit besonders 95 bis 100 Gew.-%ig) eingesetzt.

Das Edukt BDO kann als Lösung, z.B. als wässrige Lösung, besonders als 75 bis 95 Gew.-%ige wässrige Lösung, oder ohne Lösungsmittel eingesetzt werden. Ganz besonders wird es ohne Lösungsmittel (bevorzugte Reinheit 95 bis 100 Gew.-%ig, besonders 98 bis 100 Gew.-%ig) eingesetzt.

Bevorzugt wird eine Abgasmenge von 1 bis 450 Normkubikmeter / (m³ Katalysator (Schüttvolumen) • h), insbesondere 2 bis 200 Normkubikmeter / (m³ Katalysator (Schüttvolumen) • h), gefahren.

[Normkubikmeter = auf Normalbedingungen (20 °C, 1 bar abs.) umgerechnetes Volumen]. Katalysatorvolumen-Angaben beziehen sich immer auf das Schüttvolumen.

Die Aminierung der primären Alkoholgruppen des Edukts BDO wird in der Flüssigphase durchgeführt. Bevorzugt ist das Festbettverfahren in der Flüssigphase.

Beim Arbeiten in der Flüssigphase leitet man die Edukte (BDO, Ammoniak), bevorzugt simultan, in flüssiger Phase bei Drücken von 16,0 bis 22,0 MPa (160 bis 220 bar), bevorzugt 17,0 bis 21,0 MPa, weiter bevorzugt 18,0 bis 20,0 MPa, weiter bevorzugt 18,5 bis 19,5 MPa, und Temperaturen von 160 bis 230 °C, besonders 165 bis 220 °C, bevorzugt 170 bis 215 °C, insbesondere 175 bis 210 °C, weiter besonders 180 bis 200 °C, weiter ganz besonders 180 bis 195 °C, z.B. 180 bis 190 °C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Rieselfahrweise ist bevorzugt. Die Katalysatorbelastung liegt im Allgemeinen im Bereich von 0,1 bis 0,7, bevorzugt 0,15 bis 0,6, besonders bevorzugt 0,2 bis 0,5, kg BDO pro Liter Katalysator (Schüttvolumen) und Stunde (BDO berechnet als 100 Gew.-%ig). Gegebenenfalls kann eine Verdünnung der Edukte mit einem geeigneten Lösungsmittel, wie Wasser, Tetrahydrofuran, Dioxan, N-Methylpyrrolidon oder Ethylenglykoldimethylether, erfolgen. Es ist zweckmäßig, die Reaktanden bereits vor der Zuführung in das Reaktionsgefäß zu erwärmen, und zwar bevorzugt auf die Reaktionstemperatur.

Die Umsetzung wird in Gegenwart von 1,0 bis 4,5 Gew.-% Wasserstoff, besonders in Gegenwart von 1,2 bis 4,5 Gew.-% Wasserstoff, weiter besonders in Gegenwart von 1,5 bis 4,0 Gew.-% Wasserstoff, ganz besonders in Gegenwart von 2,0 bis 4,0 Gew.-% Wasserstoff, weiter ganz besonders in Gegenwart von 2,5 bis 3,7 Gew.-% Wasserstoff, jeweils bezogen auf die Menge an eingesetztem BDO, durchgeführt.

Der Wasserstoff wird der Reaktion bevorzugt in einer Menge im Bereich von 10 bis 45 Normliter pro Mol BDO, besonders 12 bis 45 Normliter pro Mol BDO, 15 bis 40 Normliter pro Mol BDO, 20 bis 40 Normliter pro Mol BDO, ganz besonders 25 bis 37 Normliter pro Mol BDO, (jeweils gemessen am Reaktoreingang) zugeführt.
[Normliter = NI = auf Normalbedingungen (20 °C, 1 bar abs.) umgerechnetes Volumen].
(Anmerkung: 1,0 bis 4,5 Gew.-% Wasserstoff, bezogen auf die Menge an eingesetztem BDO, entspricht einer Menge von 10 bis 45 NI H₂ pro mol BDO).

Der Druck im Reaktionsgefäß, welcher sich aus der Summe der Partialdrücke des Ammoniaks, des BDOs und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Bei dem kontinuierlichen Arbeiten in der Flüssigphase kann der überschüssige Ammoniak zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte
Alkoholgruppe) wirkt sich im Allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z. B. durch eine fraktionierende Rektifikation. Geeignete Aufarbeitungsverfahren sind z. B. in EP 1 312 600 A und EP 1 312 599 A (beide BASF AG) beschrieben. Der überschüssige Ammoniak und der Wasserstoff werden vorteilhaft wieder in die Reaktionszone zurückgeführt. Das gleiche gilt für die eventuell nicht vollständig umgesetztes BDO.

Eine Aufarbeitung des Produkts der Umsetzung ist bevorzugt wie folgt ausgestaltet (Schritte i - vi):
(i) zunächst wird unumgesetzter Ammoniak durch Destillation über Kopf abgetrennt,
(ii) das Verfahrensprodukt Pyrrolidin (I) wird zusammen mit Wasser und ggf. vorhandenen Nebenprodukten mit einem niedrigeren Siedepunkt als dem des Verfahrensprodukts I (Niedersieder) durch Destillation über Kopf abgetrennt, wobei ggf. vorhandene Nebenprodukte mit einem höheren Siedepunkt als dem des Verfahrensprodukts I (Hochsieder) und ggf. vorhandenes unumgesetztes BDO (II) im Sumpf verbleiben,
(iii) das Pyrrolidin (I) wird in einer Extraktionskolonne entwässert,
(iv) aus dem resultierenden Roh-Pyrrolidin werden Rest-Wassermengen durch Azeotropdestillation mit Pyrrolidin über Kopf abgetrennt,
(v) das resultierende Pyrrolidin wird durch Destillation über Kopf weiter gereinigt, wobei es besonders in einer Reinheit von > 97 Gew.-%, ganz besonders 98 bis 100 Gew.-%, anfällt.

Nebenprodukte mit einem höheren Siedepunkt als dem des Verfahrensprodukts I (Hochsieder) sind z.B. Verbindungen mit den folgenden Formeln:

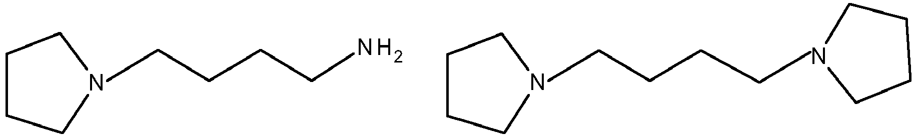

Bei der Umsetzung des erfindungsgemäßen Verfahrens können als Nebenprodukte das 4-Amino-1-butanol mit der Formel III 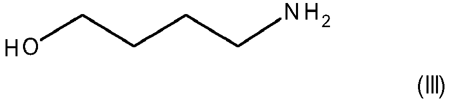 und auch das 1,4-Diamino-butan mit der Formel IV 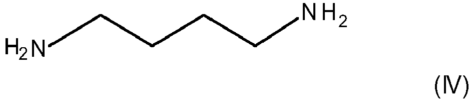 entstehen, die beide jeweils unter Ringschluss (und Freisetzung von H₂O bzw. NH₃) weiter zu Pyrrolidin (I) umgesetzt werden können.

Deshalb werden im Besonderen durch Destillation
(vi) aus dem Sumpf des Schrittes ii ggf. vorhandenes unumgesetztes BDO (II) und/oder ggf. vorhandenes 4-Amino-1-butanol als Nebenprodukt mit der Formel III und/oder ggf. vorhandenes 1,4-Diamino-butan als Nebenprodukt mit der Formel IV über Kopf abgetrennt und in die Umsetzung zurückgeführt.

In Schritt i abgetrennter Ammoniak, besonders mit einer Reinheit von 90 bis 99,9 Gew.-%, weiter besonders 95 bis 99,9 Gew.-%, wird bevorzugt in die Umsetzung zurückgeführt, wobei in einer besonderen Ausgestaltung ein Teil des abgetrennten Ammoniaks, besonders 1 bis 30 Gew.-% des abgetrennten Ammoniaks, weiter besonders 2 bis 20 Gew.-% des abgetrennten Ammoniaks, ausgeschleust wird.

In Schritt iv abgetrenntes Wasser-Pyrrolidin-Azeotrop wird bevorzugt in die Extraktionskolonne (Schritt iii) zurückgeführt.

Alle Druckangaben beziehen sich auf den Absolutdruck.
Alle ppm-Angaben beziehen sich auf die Masse.

### Beispiele

### 1. Herstellung des Katalysators A [= Beispiel 4 in WO 2011/067199 A (BASF SE)]

Eine wässrige Lösung aus Nickelnitrat, Kobaltnitrat, Kupfernitrat, Aluminiumnitrat, und Zinn(II)chlorid, die 3,9 Gew.-% Ni, 3,9 Gew.-% Co, 1,9 Gew.-% Cu, 5,5 Gew.-% Al₂O₃ und 0,5 Gew.-% Sn enthielt, wurde gleichzeitig in einem Rührgefäß in einem konstanten Strom mit einer 20 Gew.-%igen wässrigen Natriumcarbonatlösung bei einer Temperatur von 65-70 °C so gefällt, dass der mit einer Glaselektrode gemessene pH-Wert von 5,7 aufrechterhalten wurde. Nach der Fällung wurde für 1 Stunde Luft eingeblasen, danach wurde das pH des Lösungs mit Natriumcarbonatlösung auf den Wert 7,4 eingestellt. Die erhaltene Suspension wurde filtriert und der Filterkuchen mit vollentsalztem Wasser gewaschen bis die elektrische Leitfähigkeit des Filtrats ca. 20 mS betrug. Danach wurde der Filterkuchen bei einer Temperatur von 150 °C in einem Trockenschrank getrocknet. Das auf diese Weise erhaltene Hydroxidcarbonatgemisch wurde nun bei einer Temperatur von 500 °C über 4 Stunden calciniert. Die Katalysatormasse wurde anschließend mit 3 Gew.-% Graphit vermischt und zu Tabletten 3x3 mm verformt. Die auf diese Weise erhaltenen Tabletten werden bei einer Temperatur von 280-300 °C über mindestens 12 Stunden in Wasserstoff reduziert. Die Passivierung des reduzierten Katalysators wurde bei Raumtemperatur in verdünnter Luft (Luft in N₂ mit einem O₂-Gehalt von maximal 5 Vol.-%) durchgeführt. Der so erhaltene Katalysator hatte die Zusammensetzung wie in der folgenden Tabelle I dargestellt.

**Tabelle I**

| Katalysator *) | Ni | Co | Cu | Sn | BET**) | Träger |
|---|---|---|---|---|---|---|
| | % | % | % | % | m²/g | |
| Katalysator A | 18,6 | 17,3 | 10,6 | 1,1 | 187 | Al₂O₃ |

| | | | | | | |
|---|---|---|---|---|---|---|
| *) Katalysatorzusammensetzung in Gew.-%; Rest bis zu 100 Gew.-% ist der Träger **) ISO 9277:1995 | | | | | | |

### 2. Umsetzung von BDO mit Ammoniak in Rieselfahrweise in einem kontinuierlich betriebenen Rohrreaktor (Schachtreaktor)

Ein beheizter Rohrreaktor mit 14 mm Innendurchmesser, einem zentral angebrachten Thermoelement und einem Gesamtvolumen von 1000 ml wurde im unteren Teil mit einer Schicht Glaskugeln (250 ml) befüllt, darüber mit 500 ml des reduzierten Katalysators A und schließlich der restliche Teil wiederum mit Glaskugeln befüllt. Vor der Reaktion wurde der Katalysator bei max. 280 °C unter Wasserstoff (25 NI / h) [NI = Normliter = auf Normalbedingungen (20 °C, 1 bar abs.) umgerechnetes Volumen] bei Normaldruck 24 Stunden aktiviert. Durch den Reaktor wurden von oben nach unten eine bestimmte Menge BDO (99 Gew.-%ige Schmelze), Ammoniak und Wasserstoff, wie in der folgenden Tabelle II angegeben, dosiert. Der Reaktor wurde bei einer Temperatur von ca. 178 bis 184 °C (siehe Tabelle) und einem Gesamtdruck von 190 bar gehalten. Die Reaktionstemperatur wurde so gewählt, dass das BDO voll umgesetzt wurde. Das aus dem Reaktor austretende Gemisch wurde abgekühlt und auf Normaldruck entspannt. Zu verschiedenen Zeitpunkten wurden Proben vom Reaktionsgemisch genommen und mittels Gaschromatographie analysiert. Hierfür wurde eine 30 m lange GC Säule "RTX-5 Amine" verwendet, mit einem Temperaturprogramm: 70 °C/5 Min., aufheizen auf 280 °C mit einer Geschwindigkeit von 5 °C/Min., bei 280 °C/10 Minuten.

Die Ergebnisse der Versuche sind der folgenden Tabelle II zu entnehmen

**Tabelle II**

| Beispiel | Temp. | Bel. BDO | MV | Wasserstoff | | | | | BDO Umsatz | PYR-Sel. | Einsatzzahl | Kapazität |
|---|---|---|---|---|---|---|---|---|---|---|---|---|
| | | | | (1) | (2) | (3) | (4) | (5) | | | | |
| B1 | 183 | 0,26 | 20 | 13,5 | 27 | 79 | 0,4 | 2,71 | 100 | 70,3 | 180 | 146 |
| VB1a | 182 | 0,26 | 20 | 40,5 | 81 | 236 | 1,2 | 8,10 | 100 | 68,1 | 186 | 142 |
| VB1b | 178 | 0,26 | 20 | 90 | 180 | 526 | 2,6 | 18,06 | 100 | 58,0 | 218 | 120 |
| B2 | 183 | 0,26 | 25 | 13,5 | 27 | 79 | 0,4 | 2,71 | 100 | 74,0 | 171 | 154 |
| VB2 | 184 | 0,26 | 25 | 40,5 | 81 | 236 | 1,2 | 8,10 | 100 | 71,4 | 177 | 148 |
| B3 | 180 | 0,20 | 33 | 18 | 36 | 79 | 0,4 | 3,53 | 100 | 78,4 | 162 | 122 |
| VB3 | 180 | 0,20 | 33 | 54 | 108 | 236 | 1,2 | 10,54 | 100 | 75,5 | 168 | 118 |

| | | | | | | | | | | | | |
|---|---|---|---|---|---|---|---|---|---|---|---|---|
| Druck: 190 bar Beispiele: Erfindungsgemäße Beispiele (B); Vergleichsbeispiele (VB) Temp.: Temperatur im Reaktor in °C Bel. BDO: Belastung Butandiol in kg pro Liter Katalysator und Stunde MV: Molverhältnis NH₃ : BDO Wasserstoff: angegeben in 4 Einheiten: (1) Normliter pro Mol Alkoholfunktion; (2) Normliter pro Mol BDO; (3) Normkubikmeter pro Kubikmeter Katalysator und Stunde; (4) Betriebskubikmeter (bei Betriebsdruck) pro Kubikmeter Katalysator und Stunde (5) Gew.-% bezogen auf die Menge an eingesetztem BDO BDO Umsatz: Umsatz von Butandiol in % PYR-Sel.: Selektivität von Pyrrolidin in mol% Einsatzzahl: kg Butandiol pro 100 kg Pyrrolidin Kapazität: kg Pyrrolidin pro Kubikmeter Katalysator und Stunde | | | | | | | | | | | | |

Die Aufarbeitung kann bevorzugt durch die folgenden Schritte erfolgen:
Ausübung des Verfahrens mit Kreisgas (Kreisgasverfahren): Nach dem Durchgang durch den Reaktor wird das Gemisch abgekühlt und die Gas- von der Flüssigphase abgetrennt. Die Gasphase, die hauptsächlich aus Wasserstoff und teilweise aus Ammoniak besteht, wird als Kreisgas in den Reaktor zurückgeführt. Verbrauch und Verlust an Wasserstoff wird mit Frisch-H₂-gas kompensiert, um den Druck im Reaktor konstant zu halten.
Bei Ausübung des Verfahrens ohne Kreisgas wird das Frisch-H₂-gas in geradem Durchgang im Reaktor eingespeist und nach dem Hochdruckabscheider entsorgt.

Vom Flüssigaustrag wird in einer Kolonne der Ammoniak (NH₃) abgetrennt und bevorzugt in den Reaktor zurückgeführt. Ggf. wird vorteilhaft ein Teil des Ammoniaks vom Kopf der Kolonne ausgeschleust. Danach wird der Flüssigaustrag aufgearbeitet:
- In einer ersten Destillationskolonne werden Schwersieder (höher siedende Nebenkomponenten) im Sumpf abgetrennt und Pyrrolidin zusammen mit Wasser und sonstigen Leichtsiedern über Kopf abgezogen.
- In einer Extraktionskolonne wird mit 50 Gew.-%iger Natronlauge entwässert.
- In einer zweiten Destillationskolonne wird das Rest-Wasser im Pyrrolidin als Azeotrop mit Pyrrolidin über Kopf abgetrennt und bevorzugt in die Extraktionskolonne zurückgeführt.
- In einer dritten Destillationskolonne wird reines Pyrrolidin, bevorzugt in einer Reinheit von 98 bis 100 Gew.-%, abdestilliert.

## Patentansprüche

1. Verfahren zur Herstellung von Pyrrolidin der Formel I 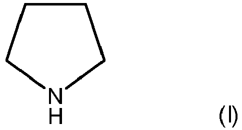 durch Umsetzung von 1,4-Butandiol (BDO) der Formel II 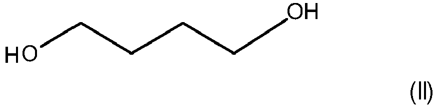 mit Ammoniak in Gegenwart von Wasserstoff und eines geträgerten, metallhaltigen Katalysators, **dadurch gekennzeichnet, dass** die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthält und man die Umsetzung in der Flüssigphase bei einem Absolutdruck im Bereich von 160 bis 220 bar, einer Temperatur im Bereich von 160 bis 230 °C, unter Einsatz von Ammoniak in einem Molverhältnis zu eingesetztem BDO von 5 bis 50 und in Gegenwart von 1,0 bis 4,5 Gew.-% Wasserstoff, bezogen auf die Menge an eingesetztem BDO, durchführt.

2. Verfahren nach Anspruch 1, **dadurch gekennzeichnet, dass** die katalytisch aktive Masse des Katalysators vor dessen Reduktion mit Wasserstoff im Bereich von 0,4 bis 4,0 Gew.-% sauerstoffhaltige Verbindungen des Zinns, berechnet als SnO, enthält.

3. Verfahren nach Anspruch 1, **dadurch gekennzeichnet, dass** die katalytisch aktive Masse des Katalysators vor dessen Reduktion mit Wasserstoff im Bereich von 0,6 bis 3,0 Gew.-% sauerstoffhaltige Verbindungen des Zinns, berechnet als SnO, enthält.

4. Verfahren nach einem der vorhergehenden Ansprüche, **dadurch gekennzeichnet, dass** die katalytisch aktive Masse des Katalysators vor dessen Reduktion mit Wasserstoff im Bereich von 5,0 bis 35 Gew.-% sauerstoffhaltige Verbindungen des Kobalts, berechnet als CoO, enthält.

5. Verfahren nach einem der Ansprüche 1 bis 3, **dadurch gekennzeichnet, dass** die katalytisch aktive Masse des Katalysators vor dessen Reduktion mit Wasserstoff im Bereich von 10 bis 30 Gew.-% sauerstoffhaltige Verbindungen des Kobalts, berechnet als CoO, enthält.

6. Verfahren nach einem der vorhergehenden Ansprüche, **dadurch gekennzeichnet, dass** die katalytisch aktive Masse des Katalysators vor dessen Reduktion mit Wasserstoff im Bereich von 15 bis 80 Gew.-% sauerstoffhaltige Verbindungen des Aluminiums, berechnet als Al₂O₃, 1,0 bis 20 Gew.-% sauerstoffhaltige Verbindungen des Kupfers, berechnet als CuO, und 5,0 bis 35 Gew.-% sauerstoffhaltige Verbindungen des Nickels, berechnet als NiO, enthält.

7. Verfahren nach einem der Ansprüche 1 bis 5, **dadurch gekennzeichnet, dass** die katalytisch aktive Masse des Katalysators vor dessen Reduktion mit Wasserstoff im Bereich von 30 bis 70 Gew.-% sauerstoffhaltige Verbindungen des Aluminiums, berechnet als Al₂O₃, 2,0 bis 18 Gew.-% sauerstoffhaltige Verbindungen des Kupfers, berechnet als CuO, und 10 bis 30 Gew.-% sauerstoffhaltige Verbindungen des Nickels, berechnet als NiO, enthält.

8. Verfahren nach einem der vorhergehenden Ansprüche, **dadurch gekennzeichnet, dass** im Katalysator das Molverhältnis von Nickel zu Kupfer größer 1 ist.

9. Verfahren nach einem der vorhergehenden Ansprüche, **dadurch gekennzeichnet, dass** die katalytisch aktive Masse des Katalysators kein Rhenium und/oder Ruthenium enthält.

10. Verfahren nach einem der vorhergehenden Ansprüche, **dadurch gekennzeichnet, dass** die katalytisch aktive Masse des Katalysators kein Eisen und/oder Zink enthält.

11. Verfahren nach einem der vorhergehenden Ansprüche, **dadurch gekennzeichnet, dass** die katalytisch aktive Masse des Katalysators keine sauerstoffhaltigen Verbindungen des Siliziums und/oder des Zirkoniums und/oder des Titans enthält.

12. Verfahren nach einem der vorhergehenden Ansprüche, **dadurch gekennzeichnet, dass** die BET-Oberfläche des Katalysators (ISO 9277:1995) im Bereich von 30 bis 250 m²/g beträgt.

13. Verfahren nach einem der vorhergehenden Ansprüche, **dadurch gekennzeichnet, dass** man die Umsetzung bei einer Temperatur im Bereich von 165 bis 220 °C durchführt.

14. Verfahren nach einem der vorhergehenden Ansprüche, **dadurch gekennzeichnet, dass** man die Umsetzung bei einem Absolutdruck im Bereich von 170 bis 210 bar durchführt.

15. Verfahren nach einem der vorhergehenden Ansprüche, **dadurch gekennzeichnet, dass** der Ammoniak in der 11- bis 45-fachen molaren Menge bezogen auf das eingesetzte BDO eingesetzt wird.

16. Verfahren nach einem der vorhergehenden Ansprüche, **dadurch gekennzeichnet, dass** man die Umsetzung in Gegenwart von 1,2 bis 4,5 Gew.-% Wasserstoff, bezogen auf die Menge an eingesetztem BDO, durchführt.

17. Verfahren nach einem der vorhergehenden Ansprüche, **dadurch gekennzeichnet, dass** der Katalysator im Reaktor als Festbett angeordnet ist.

18. Verfahren nach einem der vorhergehenden Ansprüche, **dadurch gekennzeichnet, dass** die Umsetzung bei einer Rieselfahrweise erfolgt.

19. Verfahren nach einem der vorhergehenden Ansprüche, **dadurch gekennzeichnet, dass** es kontinuierlich durchgeführt wird.

20. Verfahren nach dem vorhergehenden Anspruch, **dadurch gekennzeichnet, dass** die Umsetzung in einem Rohrreaktor erfolgt.

21. Verfahren nach einem der beiden vorhergehenden Ansprüche, **dadurch gekennzeichnet, dass** die Umsetzung bei einer Kreisgasfahrweise erfolgt.

22. Verfahren nach einem der vorhergehenden Ansprüche, **dadurch gekennzeichnet, dass** man das BDO lösungsmittelfrei einsetzt.

23. Verfahren nach einem der vorhergehenden Ansprüche, **dadurch gekennzeichnet, dass** man den Ammoniak als wässrige Lösung einsetzt.

24. Verfahren nach einem der vorhergehenden Ansprüche, **dadurch gekennzeichnet, dass** man die Umsetzung bei einer Katalysatorbelastung im Bereich von 0,1 bis 0,7 kg BDO / (l_{kat.} • h) durchführt.

25. Verfahren nach einem der vorhergehenden Ansprüche, **dadurch gekennzeichnet, dass** das Reaktionsprodukt der Umsetzung durch die folgenden Schritte aufgearbeitet wird:
(i) zunächst wird unumgesetzter Ammoniak durch Destillation über Kopf abgetrennt,
(ii) das Verfahrensprodukt Pyrrolidin (I) wird zusammen mit Wasser und ggf. vorhandenen Nebenprodukten mit einem niedrigeren Siedepunkt als dem des Verfahrensprodukts I durch Destillation über Kopf abgetrennt, wobei ggf. vorhandene Nebenprodukte mit einem höheren Siedepunkt als dem des Verfahrensprodukts I und ggf. vorhandenes unumgesetztes BDO (II) im Sumpf verbleiben,
(iii) das Pyrrolidin (I) wird in einer Extraktionskolonne entwässert,
(iv) aus dem resultierenden Roh-Pyrrolidin werden Rest-Wassermengen durch Azeotropdestillation mit Pyrrolidin über Kopf abgetrennt,
(v) das resultierende Pyrrolidin wird durch Destillation über Kopf weiter gereinigt.

26. Verfahren nach dem vorhergehenden Anspruch, **dadurch gekennzeichnet, dass** durch Destillation
(vi) aus dem Sumpf des Schrittes ii ggf. vorhandenes unumgesetztes BDO (II) und/oder ggf. vorhandenes 4-Amino-1-butanol als Nebenprodukt mit der Formel III 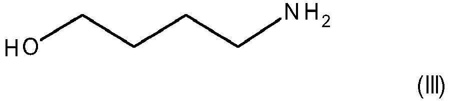 und/oder ggf. vorhandenes 1,4-Diamino-butan als Nebenprodukt mit der Formel IV 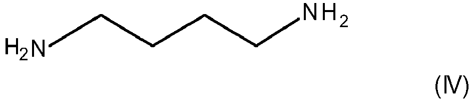 über Kopf abgetrennt und in die Umsetzung zurückgeführt werden.

27. Verfahren nach einem der beiden vorhergehenden Ansprüche, **dadurch gekennzeichnet, dass** in Schritt i abgetrennter Ammoniak mit einer Reinheit von 90 bis 99,9 Gew.-% in die Umsetzung zurückgeführt wird.

28. Verfahren nach einem der drei vorhergehenden Ansprüche, **dadurch gekennzeichnet, dass** in Schritt iv abgetrenntes Wasser-Pyrrolidin-Azeotrop in die Extraktionskolonne (Schritt iii) zurückgeführt wird.

## Claims

1. A process for preparing pyrrolidine of the formula I 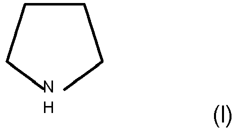 by reacting 1,4-butanediol (BDO) of the formula II 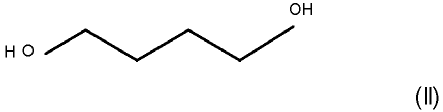 with ammonia in the presence of hydrogen and a supported, metal-containing catalyst, wherein the catalytically active mass of the catalyst, prior to its reduction with hydrogen, comprises oxygen-containing compounds of aluminum, copper, nickel and cobalt and in the range from 0.2 to 5.0% by weight of oxygen-containing compounds of tin, calculated as SnO, and the reaction is carried out in the liquid phase at an absolute pressure in the range from 160 to 220 bar, a temperature in the range from 160 to 230°C, using ammonia in a molar ratio to BDO used of from 5 to 50 and in the presence of 1.0 to 4.5% by weight of hydrogen, based on the amount of BDO used.

2. The process according to claim 1, wherein the catalytically active mass of the catalyst, prior to its reduction with hydrogen, comprises in the range from 0.4 to 4.0% by weight of oxygen-containing compounds of tin, calculated as SnO.

3. The process according to claim 1, wherein the catalytically active mass of the catalyst, prior to its reduction with hydrogen, comprises in the range from 0.6 to 3.0% by weight of oxygen-containing compounds of tin, calculated as SnO.

4. The process according to any one of the preceding claims, wherein the catalytically active mass of the catalyst, prior to its reduction with hydrogen, comprises in the range from 5.0 to 35% by weight of oxygen-containing compounds of cobalt, calculated as CoO.

5. The process according to any one of claims 1 to 3, wherein the catalytically active mass of the catalyst, prior to its reduction with hydrogen, comprises in the range from 10 to 30% by weight of oxygen-containing compounds of cobalt, calculated as CoO.

6. The process according to any one of the preceding claims, wherein the catalytically active mass of the catalyst, prior to its reduction with hydrogen, comprises in the range from
15 to 80% by weight of oxygen-containing compounds of aluminum, calculated as Al₂O₃,
1.0 to 20% by weight of oxygen-containing compounds of copper, calculated as CuO, and
5.0 to 35% by weight of oxygen-containing compounds of nickel, calculated as NiO.

7. The process according to any one of claims 1 to 5, wherein the catalytically active mass of the catalyst, prior to its reduction with hydrogen, comprises in the range from
30 to 70% by weight of oxygen-containing compounds of aluminum, calculated as Al₂O₃,
2.0 to 18% by weight of oxygen-containing compounds of copper, calculated as CuO, and
10 to 30% by weight of oxygen-containing compounds of nickel, calculated as NiO.

8. The process according to any one of the preceding claims, wherein the molar ratio of nickel to copper in the catalyst is greater than 1.

9. The process according to any one of the preceding claims, wherein the catalytically active mass of the catalyst comprises no rhenium and/or ruthenium.

10. The process according to any one of the preceding claims, wherein the catalytically active mass of the catalyst comprises no iron and/or zinc.

11. The process according to any one of the preceding claims, wherein the catalytically active mass of the catalyst comprises no oxygen-containing compounds of silicon and/or of zirconium and/or of titanium.

12. The process according to any one of the preceding claims, wherein the BET surface area of the catalyst (ISO 9277:1995) is in the range from 30 to 250 m²/g.

13. The process according to any one of the preceding claims, wherein the reaction is carried out at a temperature in the range from 165 to 220°C.

14. The process according to any one of the preceding claims, wherein the reaction is carried out at an absolute pressure in the range from 170 to 210 bar.

15. The process according to any one of the preceding claims, wherein the ammonia is used in an 11- to 45-fold molar amount, based on the BDO used.

16. The process according to any one of the preceding claims, wherein the reaction is carried out in the presence of from 1.2 to 4.5% by weight of hydrogen, based on the amount of BDO used.

17. The process according to any one of the preceding claims, wherein the catalyst is arranged as a fixed bed in the reactor.

18. The process according to any one of the preceding claims, wherein the reaction takes place in a trickle mode.

19. The process according to any one of the preceding claims, which is carried out continuously.

20. The process according to the preceding claim, wherein the reaction takes place in a tubular reactor.

21. The process according to one of the two preceding claims, wherein the reaction takes place in a circulating-gas mode.

22. The process according to any one of the preceding claims, wherein the BDO is used solvent-free.

23. The process according to any one of the preceding claims, wherein the ammonia is used as aqueous solution.

24. The process according to any one of the preceding claims, wherein the reaction is carried out at a catalyst hourly space velocity in the range from 0.1 to 0.7 kg of BDO / (l_{cat.} • h).

25. The process according to any one of the preceding claims, wherein the reaction product of the reaction is worked up by the following steps:
(i) firstly unreacted ammonia is separated off overhead by distillation,
(ii) the process product pyrrolidine (I) is separated off overhead by distillation together with water and any by-products present having a lower boiling point than that of the process product I, with any by-products present having a higher boiling point than that of process product I and any unreacted BDO (II) present remaining in the bottom,
(iii) the pyrrolidine (I) is dewatered in an extraction column,
(iv) residual amounts of water are separated off overhead from the resulting crude pyrrolidine by azeotropic distillation with pyrrolidine,
(v) the resulting pyrrolidine is further purified overhead by distillation.

26. The process according to the preceding claim, wherein, by distillation,
(vi) from the bottom of step ii, any unreacted BDO (II) present and/or any 4-amino-1-butanol present as by-product with the formula III 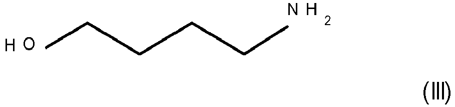 and/or any 1,4-diaminobutane present as by-product with the formula IV 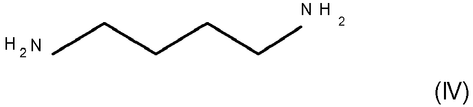 are separated off overhead and returned to the reaction.

27. The process according to one of the two preceding claims, wherein ammonia separated off in step i and having a purity of from 90 to 99.9% by weight is returned to the reaction.

28. The process according to any one the three preceding claims, wherein water-pyrrolidine azeotrope separated off in step iv is returned to the extraction column (step iii).

## Revendications

1. Procédé de fabrication de pyrrolidine de formule I 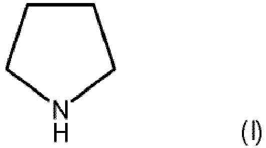 par mise en réaction de 1,4-butanediol (BDO) de formule II 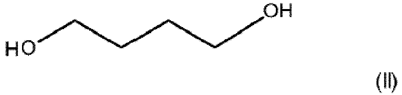 avec de l'ammoniac en présence d'hydrogène et d'un catalyseur supporté contenant un métal, **caractérisé en ce que** la masse catalytiquement active du catalyseur contient avant sa réduction avec de l'hydrogène des composés oxygénés d'aluminium, de cuivre, de nickel et de cobalt et dans la plage allant de 0,2 à 5,0 % en poids de composés oxygénés d'étain, calculés en tant que SnO, et la réaction est réalisée en phase liquide à une pression absolue dans la plage allant de 160 à 220 bar, à une température dans la plage allant de 160 à 230 °C, en utilisant de l'ammoniac en un rapport molaire par rapport au BDO utilisé de 5 à 50, et en présence de 1,0 à 4,5 % en poids d'hydrogène, par rapport à la quantité de BDO utilisé.

2. Procédé selon la revendication 1, **caractérisé en ce que** la masse catalytiquement active du catalyseur contient avant sa réduction avec de l'hydrogène dans la plage allant de 0,4 à 4,0 % en poids de composés oxygénés d'étain, calculés en tant que SnO.

3. Procédé selon la revendication 1, **caractérisé en ce que** la masse catalytiquement active du catalyseur contient avant sa réduction avec de l'hydrogène dans la plage allant de 0,6 à 3,0 % en poids de composés oxygénés d'étain, calculés en tant que SnO.

4. Procédé selon l'une quelconque des revendications précédentes, **caractérisé en ce que** la masse catalytiquement active du catalyseur contient avant sa réduction avec de l'hydrogène dans la plage allant de 5,0 à 35 % en poids de composés oxygénés de cobalt, calculés en tant que CoO.

5. Procédé selon l'une quelconque des revendications 1 à 3, **caractérisé en ce que** la masse catalytiquement active du catalyseur contient avant sa réduction avec de l'hydrogène dans la plage allant de 10 à 30 % en poids de composés oxygénés de cobalt, calculés en tant que CoO.

6. Procédé selon l'une quelconque des revendications précédentes, **caractérisé en ce que** la masse catalytiquement active du catalyseur contient avant sa réduction avec de l'hydrogène dans la plage allant de 15 à 80 % en poids de composés oxygénés d'aluminium, calculés en tant qu'Al₂O₃,
1,0 à 20 % en poids de composés oxygénés de cuivre calculés en tant que CuO et
5,0 à 35 % en poids de composés oxygénés de nickel, calculés en tant que NiO.

7. Procédé selon l'une quelconque des revendications 1 à 5, **caractérisé en ce que** la masse catalytiquement active du catalyseur contient avant sa réduction avec de l'hydrogène dans la plage allant de
30 à 70 % en poids de composés oxygénés d'aluminium, calculés en tant qu'Al₂O₃,
2,0 à 18 % en poids de composés oxygénés de cuivre calculés en tant que CuO et
10 à 30 % en poids de composés oxygénés de nickel, calculés en tant que NiO.

8. Procédé selon l'une quelconque des revendications précédentes, **caractérisé en ce que** le rapport molaire entre le nickel et le cuivre dans le catalyseur est supérieur à 1.

9. Procédé selon l'une quelconque des revendications précédentes, **caractérisé en ce que** la masse catalytiquement active du catalyseur ne contient pas de rhénium et/ou de ruthénium.

10. Procédé selon l'une quelconque des revendications précédentes, **caractérisé en ce que** la masse catalytiquement active du catalyseur ne contient pas de fer et/ou de zinc.

11. Procédé selon l'une quelconque des revendications précédentes, **caractérisé en ce que** la masse catalytiquement active du catalyseur ne contient pas de composés oxygénés de silicium et/ou de zirconium et/ou de titane.

12. Procédé selon l'une quelconque des revendications précédentes, **caractérisé en ce que** la surface BET du catalyseur (ISO 9277:1995) est dans la plage allant de 30 à 250 m²/g.

13. Procédé selon l'une quelconque des revendications précédentes, **caractérisé en ce que** la réaction est réalisée à une température dans la plage allant de 165 à 220 °C.

14. Procédé selon l'une quelconque des revendications précédentes, **caractérisé en ce que** la réaction est réalisée à une pression absolue dans la plage allant de 170 à 210 bar.

15. Procédé selon l'une quelconque des revendications précédentes, **caractérisé en ce que** l'ammoniac est utilisé en une quantité molaire de 11 à 45 fois par rapport au BDO utilisé.

16. Procédé selon l'une quelconque des revendications précédentes, **caractérisé en ce que** la réaction est réalisée en présence de 1,2 à 4,5 % en poids d'hydrogène, par rapport à la quantité de BDO utilisé.

17. Procédé selon l'une quelconque des revendications précédentes, **caractérisé en ce que** le catalyseur est agencé dans le réacteur sous la forme d'un lit fixe.

18. Procédé selon l'une quelconque des revendications précédentes, **caractérisé en ce que** la réaction a lieu en mode de ruissellement.

19. Procédé selon l'une quelconque des revendications précédentes, **caractérisé en ce qu'**il est réalisé en continu.

20. Procédé selon la revendication précédente, **caractérisé en ce que** la réaction a lieu dans un réacteur tubulaire.

21. Procédé selon l'une quelconque des deux revendications précédentes, **caractérisé en ce que** la réaction a lieu en mode à gaz circulaire.

22. Procédé selon l'une quelconque des revendications précédentes, **caractérisé en ce que** le BDO est utilisé sans solvant.

23. Procédé selon l'une quelconque des revendications précédentes, **caractérisé en ce que** l'ammoniac est utilisé sous la forme d'une solution aqueuse.

24. Procédé selon l'une quelconque des revendications précédentes, **caractérisé en ce que** la réaction est réalisée à un chargement du catalyseur dans la plage allant de 0,1 à 0,7 kg de BDO/(l_{cat}•h).

25. Procédé selon l'une quelconque des revendications précédentes, **caractérisé en ce que** le produit de la réaction est traité par les étapes suivantes :
(i) tout d'abord, l'ammoniac non réagi est séparé par distillation par la tête,
(ii) le produit du procédé pyrrolidine (I) est séparé conjointement avec de l'eau et des produits secondaires éventuellement présents ayant un point d'ébullition inférieur à celui du produit du procédé I par distillation par la tête, les produits secondaires éventuellement présents ayant un point d'ébullition supérieur à celui du produit du procédé I et le BDO non réagi (II) éventuellement présent restant dans le fond,
(iii) la pyrrolidine (I) est déshydratée dans une colonne d'extraction,
(iv) les quantités résiduelles d'eau sont séparées de la pyrrolidine brute résultante par distillation azéotropique avec de la pyrrolidine par la tête,
(v) la pyrrolidine résultante est davantage purifiée par distillation par la tête.

26. Procédé selon la revendication précédente, **caractérisé en ce que**, par distillation
(vi) le BDO non réagi (II) éventuellement présent et/ou le 4-amino-1-butanol éventuellement présent en tant que produit secondaire de formule III 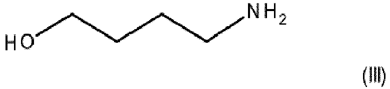 et/ou le 1,4-diamino-butane éventuellement présent en tant que produit secondaire de formule IV 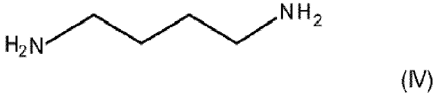 sont séparés par la tête à partir du fond de l'étape ii, et recyclés dans la réaction.

27. Procédé selon l'une quelconque des deux revendications précédentes, **caractérisé en ce que** l'ammoniac séparé à l'étape i avec une pureté de 90 à 99,9 % en poids est recyclé dans la réaction.

28. Procédé selon l'une quelconque des trois revendications précédentes, **caractérisé en ce que** l'azéotrope eau-pyrrolidine séparé à l'étape iv est recyclé dans la colonne d'extraction (étape iii).
